# EUROPEAN PATENT APPLICATION

(11) **EP 3 799 817 A1**
(43) Date of publication of application: **07.04.2021**
(21) Application number: 20200122.8
(22) Date of filing: 05.10.2020
(51) Int. Cl.: A61B 18/14, A61B 90/00, A61B 17/285, A61B 17/29

(54) **SURGICAL INSTRUMENTS FOR PERFORMING TONSILLECTOMY, ADENOIDECTOMY, AND OTHER SURGICAL PROCEDURES**

(30) Priority: 06.10.2019 US 201916594031
(71) Applicant: Covidien LP, Mansfield, MA 02048 (US)
(72) Inventor: STAMM, Stephen, J, Wheat Ridge, CO Colorado 80033 (US); SAHA, Subhadeep, Boulder, CO Colorado 80301 (US); SONI, Purvishkumar, H, Longmont, CO Colorado 80504 (US); Olson, Jessica, E.C, Frederick, CO Colorado 80504 (US); SAWYER, Alyssa, M, Boulder, CO Colorado 80301 (US)
(74) Representative: Maschio & Soames IP Ltd

(57) **Abstract**

A surgical instrument includes a housing having a rounded barrel with a distal end and proximal end configured to seat within a palm of a user. A shaft extends from the distal end of the barrel and supports an end effector at a distal end thereof including first and second jaw members. A rotating assembly is disposed on the barrel and is actuatable to rotate the jaw members. A jaw actuation assembly is disposed on the barrel and is actuatable to move the jaw members between open and closed positions. An energy activation assembly is disposed on the barrel and is actuatable to supply electrosurgical energy to the jaw members. A knife actuation assembly is disposed on the barrel and is actuatable to cut tissue disposed between the jaw members.

## Description

### BACKGROUND

### TECHNICAL FIELD

The present disclosure relates to surgical instruments and, more particularly, to surgical instruments for performing tonsillectomy, adenoidectomy, and other surgical procedures.

### BACKGROUND OF RELATED ART

The tonsils and adenoids are part of the lymphatic system and are generally located in the back of the throat. These parts of the lymphatic system are generally used for sampling bacteria and viruses entering the body and activating the immune system when warranted to produce antibodies to fight oncoming infections. More particularly, the tonsils and adenoids break down the bacteria or virus and send pieces of the bacteria or virus to the immune system to produce antibodies for fighting off infections.

Inflammation of the tonsils and adenoids (e.g., tonsillitis) impedes the ability of the tonsils and adenoids to destroy the bacteria resulting in a bacterial infection. In many instances, the bacteria remain even after treatment and serve as a reservoir for repeated infections (e.g., tonsillitis or ear infections).

A tonsillectomy and/or adenoidectomy may be performed when infections persist and antibiotic treatments fail. Some individuals are also born with larger tonsils that are more prone to cause obstruction. An adenoidectomy may also be required to remove adenoid tissue when ear pain persists, or when nose breathing or function of the Eustachian tube is impaired. Often times, tonsillectomy and adenoidectomy procedures are performed at the same time.

### SUMMARY

As used herein, the term "distal" refers to the portion that is being described which is further from a user, while the term "proximal" refers to the portion that is being described which is closer to a user. Further, to the extent consistent, any of the aspects described herein may be used in conjunction with any or all of the other aspects described herein.

A surgical instrument provided in accordance with aspects of the present disclosure includes a housing having a rounded barrel portion with a distal end portion and proximal end portion configured to seat within a palm of a user. A shaft extends from the distal end portion of the barrel portion and supports an end effector assembly at a distal end thereof configured to treat tissue. The end effector assembly includes first and second jaw members.

A rotating assembly is disposed on an outer surface of the barrel portion and is actuatable to rotate the jaw members about a longitudinal axis defined through the shaft. A jaw actuation assembly is disposed on the outer surface of the barrel portion and actuatable to move the jaw members between an open position wherein the jaw members are spaced relative to one another for manipulating tissue and a closed position for approximating tissue therebetween. An energy activation assembly is disposed on the outer surface of the barrel portion and is actuatable to supply electrosurgical energy from an electrosurgical energy source to the jaw members upon activation thereof. A knife actuation assembly is disposed on the outer surface of the barrel portion and is actuatable to cut tissue disposed between the jaw members upon actuation thereof.

In aspects according to the present disclosure, one or more of the rotating assembly, jaw actuation assembly, or knife actuation assembly includes a solenoid to facilitate actuation. In other aspects according to the present disclosure, one or more of the rotating assembly, jaw actuation assembly, or knife actuation assembly is a dial. In yet other aspects according to the present disclosure, one or more of the rotating assembly, jaw actuation assembly, energy activation assembly or knife actuation assembly is a depressible button. In still other aspects according to the present disclosure, one or more of the rotating assembly, jaw actuation assembly, or knife actuation assembly is a slide actuator.

In aspects according to the present disclosure, the energy activation assembly electrically communicates with a switch to energize the jaw members. In other aspects according to the present disclosure, the energy activation assembly includes a safety that impedes actuation of the knife actuation assembly until after successful completion of a tissue seal. In still other aspects according to the present disclosure, the rotating assembly includes a potentiometer for regulating the speed of rotation of the jaw members about the longitudinal axis.

A surgical instrument provided in accordance with aspects of the present disclosure includes a housing having a rounded barrel portion with a distal end portion and proximal end portion configured to seat within a palm of a user. A shaft extends from the distal end portion of the barrel portion and supports an end effector assembly at a distal end thereof configured to treat tissue. The end effector assembly includes first and second jaw members.

A rotating assembly is disposed on an outer surface of the barrel portion and is actuatable to rotate the jaw members about a longitudinal axis defined through the shaft. A jaw actuation assembly is disposed on the outer surface of the barrel portion and is actuatable to move the jaw members between an open position wherein the jaw members are spaced relative to one another for manipulating tissue and a closed position for approximating tissue therebetween. An energy activation assembly is disposed on the outer surface of the barrel portion and is actuatable to supply electrosurgical energy from an electrosurgical energy source to the jaw members upon activation thereof.

A knife actuation assembly is disposed on the outer surface of the barrel portion and actuatable to cut tissue disposed between the jaw members upon actuation thereof. The knife actuation assembly includes a knife blade and an actuation element. The actuation element is coupled to a first end of a linkage and a knife drive element is coupled to a second end of the linkage. Actuation of the activation element pivots the linkage to force the knife drive element distally to translate the knife blade through tissue disposed between the jaw members.

In aspects according to the present disclosure, one or more of the rotating assembly or jaw actuation assembly includes a solenoid to facilitate actuation. In other aspects according to the present disclosure, one or more of the rotating assembly or jaw actuation assembly is a dial. In still other aspects according to the present disclosure, one or more of the rotating assembly, jaw actuation assembly, or energy activation assembly is a depressible button.

In yet other aspects according to the present disclosure, the energy activation assembly electrically communicates with a switch to energize the jaw members. Still in other aspects, the energy activation assembly includes a safety that impedes actuation of the knife actuation assembly until after successful completion of a tissue seal. Yet, in other aspects according to the present disclosure, the rotating assembly includes a potentiometer for regulating the speed of rotation of the jaw members about the longitudinal axis.

### BRIEF DESCRIPTION OF THE DRAWINGS

Various aspects and features of the present disclosure are described herein with reference to the drawings wherein:
FIG. 1 is a perspective view of a surgical instrument provided in accordance with the present disclosure including a handle assembly, end effector assembly, trigger assembly and activation assembly; with jaw members of the end effector assembly of the surgical instrument disposed in a spaced-apart position;
FIGS. 2A-2D are rear, perspective, internal views of the surgical instrument of FIG. 1 showing the range of movement of a pair of jaw members of the end effector assembly and the movement of the various internal components of the handle assembly, trigger assembly and activation assembly;
FIG. 3 is a front, perspective, partially-exploded view of the surgical instrument of FIG. 1 with the jaw members disposed in the approximated position and a portion of the housing removed to illustrate the internal components thereof;
FIGS. 4A-4C are enlarged, perspective views of the jaw members of the end effector assembly shown in open and approximated positions and showing advancement of a knife blade to cut tissue disposed between jaw members;
FIG. 5 is a perspective view of another embodiment of the surgical instrument in accordance with the present disclosure; and
FIG. 6 is perspective view of yet another embodiment of the surgical instrument in accordance with the present disclosure.

### DETAILED DESCRIPTION

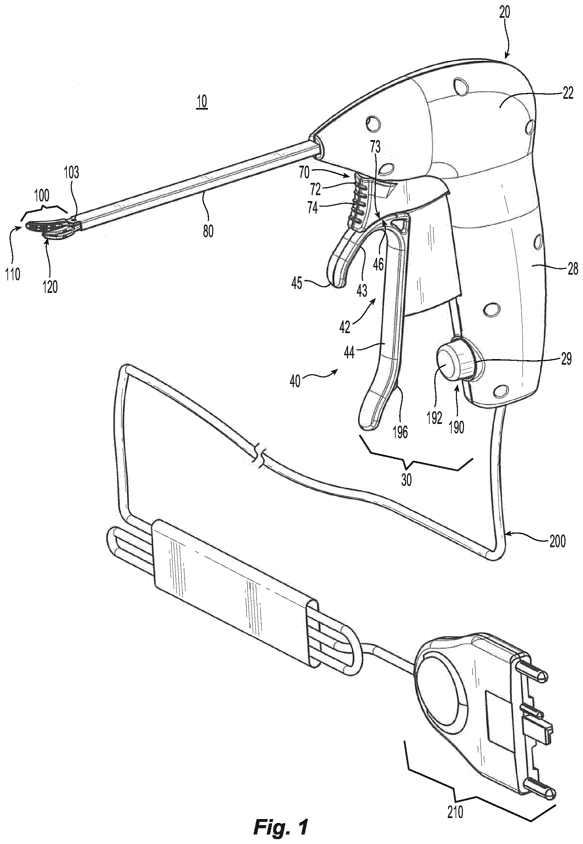

Referring generally to FIG. 1, a surgical instrument provided in accordance with the present disclosure is shown generally identified by reference numeral 10. Instrument 10, as described below, is configured for grasping, treating, and/or dissecting tissue and may find particular applicability for use in performing tonsillectomy procedures and/or adenoidectomy procedures, although use of instrument 10 in various other surgical procedures is also contemplated and within the scope of the present disclosure. Additional features contemplated for use with instrument 10 are detailed in commonly-owned U.S. Patent No. 9,918,779 issued March 20, 2018, U.S. Patent No. 9,918,780 issued March 20, 2018, U.S. Patent Publication No. 2016-0338718 published November 24, 2016, U.S. Patent No. 9,795,435 issued October 24, 2017 and U.S. Patent No. 9,918,781 issued March 20, 2018, each of which is incorporated herein by reference in its entirety.

With reference to FIGS, 1-3, instrument 10 generally includes a housing 20, a handle assembly 30, a trigger assembly 70, a shaft 80, an end effector assembly 100, a drive assembly 140, a knife assembly 170, and an energy activation assembly 190. As detailed below, shaft 80 extends distally from housing 20 and supports end effector assembly 100 at distal end of shaft 80, drive assembly 140 operably couples handle assembly 30 with end effector assembly 100 to enable selective manipulation of jaw members 110, 120 of end effector assembly 100, knife assembly 170 is operably coupled with trigger assembly 70 to enable selective translation of a knife blade 174 of knife assembly 170 relative to end effector assembly 100, and energy activation assembly 190 enables energy to be selectively delivered to end effector assembly 100.

Instrument 10 also includes an electrosurgical cable 200 including a proximal plug 210 that connects instrument 10 to a generator (not shown) or other suitable power source, although instrument 10 may alternatively be configured as a battery-powered instrument. Electrosurgical cable 200 includes lead wires extending therethrough that have sufficient length to extend through housing 20 and shaft 80 in order to operably couple the generator, energy activation assembly 190, and end effector assembly 100 with one another to enable the selective supply of energy to jaw members 110, 120 of end effector assembly 100, e.g., upon activation of activation switch 194 of energy activation assembly 190.

Housing 20 houses the internal working components of instrument 10 and is formed from first and second housing components configured to engage one another via a plurality of pin-aperture engagements spaced around housing 20, although other suitable engagements, e.g., screws, snap-fit connections, adhesion, ultrasonic welding, etc., are also contemplated, as are different formations of housing 20. Housing 20 defines a pistol-style configuration having a longitudinally-extending barrel portion 22 and a fixed handle portion 28 that extends from barrel portion 22 in generally perpendicular orientation relative thereto.

Barrel portion 22 of housing 20 defines a distal aperture configured to receive and engage the proximal end of shaft 80 therein. Shaft 80 extends distally from barrel portion 22 of housing 20 and defines a generally rectangular cross-sectional configuration oriented such that the larger width dimension thereof extends laterally and the smaller height dimension thereof extends vertically. This configuration of shaft 80 relative to the orientation of jaw members 110, 120 provides enhanced "line-of-sight" for visualizing the surgical site adjacent end effector assembly 100. Shaft 80 includes a pair of spaced-apart clevis members 84 extending from the top and bottom walls, e.g., the larger width dimension walls, of shaft 80 at the distal end of shaft 80. Each clevis member 84 defines an aperture for receiving a pivot pin 103 to operably support end effector assembly 100 at the distal end of shaft 80.

Barrel portion 22 of housing further includes a pair of opposed pivot apertures 23 (only one of which is shown), a longitudinal track 24, a pair of opposed pivot bosses 25 (only one of which is shown), and a block 26. Each pivot aperture 23 is configured to receive an end of pivot pin 48 to pivotably couple movable handle 40 and trigger 72 to housing 20. Longitudinal track 24 is configured to guide translation of drive assembly 140 relative to housing 20. Pivot bosses 25 extend inwardly into housing 20 and are configured to pivotably couple linkage 76 of trigger assembly 70 to housing 20.

Energy activation assembly 190 includes a depressible button 192 that is mechanically coupled to a switch 194 mounted within a bay 29 defined within fixed handle portion 28 of housing 20 and is engagable by a button activation post 196 extending proximally from a proximal side of movable handle 40 upon movement of movable handle 40 to the activated position, as detailed below. Switch 194 is configured to electrically communicate with end effector assembly 100 and the generator (not shown) via suitable electrical wiring to enable energy to be supplied from the generator (not shown) to end effector assembly 100 upon activation of switch 194.

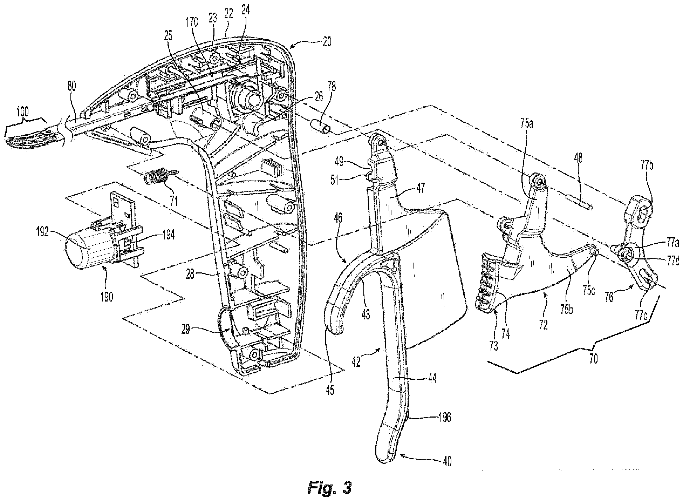

Continuing with reference to FIGS. 1-3, handle assembly 30 includes movable handle 40 that is movable relative to fixed handle portion 28 of housing 20 between an initial position, a compressed position, and an activated position, as explained in greater detail below, to impart movement of jaw members 110, 120 of end effector assembly 100 between a spaced-apart position and an approximated position for grasping tissue therebetween and for initiating the supply of energy to end effector assembly 100 for treating grasped tissue. Movable handle 40 and trigger 72 of trigger assembly 70 are ergonomically configured to facilitate manipulation and operation of instrument 10. Movable handle 40, more specifically, defines a grasping portion 42 having an arcuate segment 43 and an elongated proximal leg 44 that extends from arcuate segment 43 the length of fixed handle portion 28 of housing 20. Arcuate segment 43 culminates in a distal tail 45 and defines a sufficient diameter so as to operably receive a user's finger between distal tail 45 and proximal leg 44. Arcuate segment 43 further defines a convex surface 46. Trigger 72, more specifically, defines an abutting surface 73 that abuts convex surface 46 of arcuate segment 43 of movable handle 40 and is complementarily contoured such that, in the initial position of movable handle 40 and the un-actuated position of trigger 72, pinch points between trigger 72 and movable handle 40 are eliminated. Further, trigger 72 surrounds the exposed part of flange portion 47 of movable handle 40 to eliminate pinch points therebetween.

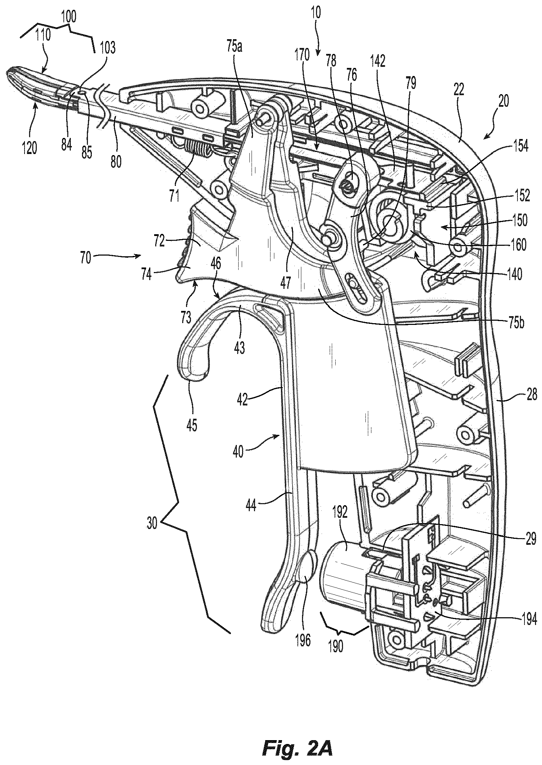

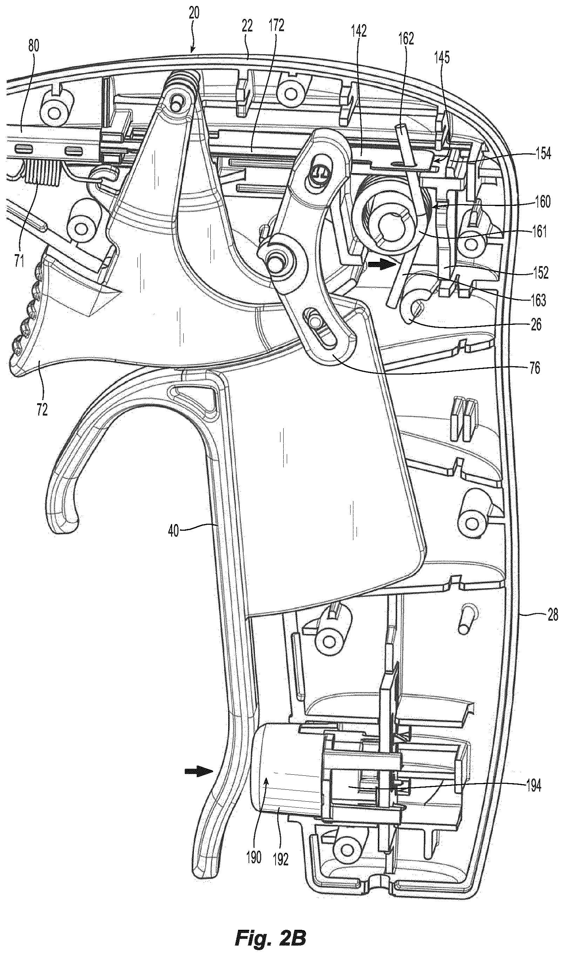

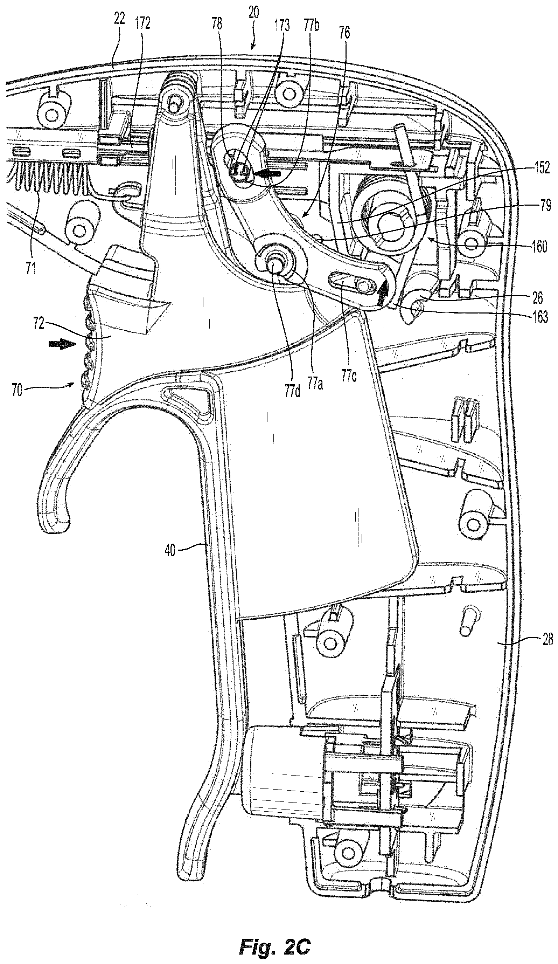

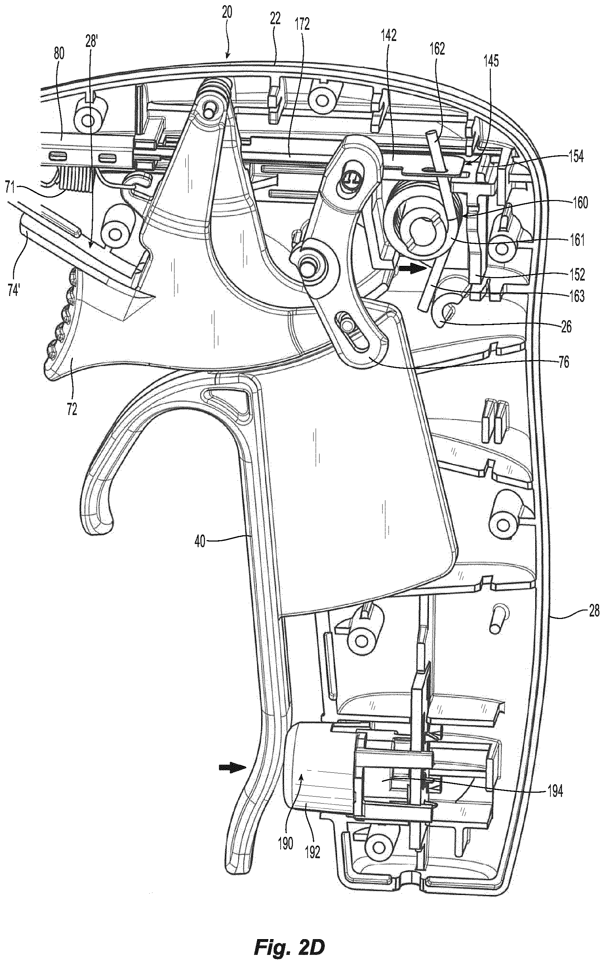

Movable handle 40 includes grasping portion 42, which extends from housing 20 adjacent fixed handle portion 28, and flange portion 47, which extends upwardly into housing 20. Flange portion 47 is pivotably coupled within housing 20 at the free end of flange portion 47 via pivot pin 48. Pivot pin 48 is engaged within and extends between pivot apertures 23 of housing 20 to permit movable handle 40 to pivot about pivot pin 48 and relative to housing 20 between the initial position (FIGS. 1 and 2), the compressed position, and the activated position.

In use, movable handle 40 is biased towards the initial position by the abutment of a lower leg 163 of a drive torsion spring (not shown) with block 26 of housing 20. With movable handle 40 in the initial position, slider assembly 150 is likewise disposed in a distal-most position. With slider assembly 150 disposed in its distal-most position, an upper leg 162 of the drive torsion spring 160 retains drive plate 142 in a distal-most position with the proximal edge 145 of drive plate 142 disposed in abutment with abutment rib 154 of proximal housing 152 of slider assembly 150. In the distal-most position of drive plate 142, drive plate 142 maintains the jaw cam pin (not shown) at the distal ends of oppositely-angled cam slots of the proximal flanges of the jaw members 110, 120 to thereby maintain jaw members 110, 120 in the spaced-apart position.

At this point, trigger 72 is disposed in the un-actuated position, wherein trigger 72 is in a distal-most position under the bias of biasing member 71 such that upper end cam slot 77b of linkage 76 is disposed in a proximal-most position while lower end cam slot 77c of linkage 76 is disposed in a distal-most position. Thus, knife plate 172 is disposed in a proximal-most position, corresponding to a retracted position of knife blade 174, wherein knife blade 174 is disposed between proximal flanges of jaw frames of jaw members 110, 120 but does not extend distally therefrom. Further, with movable handle 40 disposed in its initial position, proximal housing 152 of slider assembly 150 is disposed in the movement path of lockout peg 79 of linkage 76, inhibiting rotation of linkage 76 and, thus, inhibiting movement of trigger 72 from the un-actuated position to the actuated position. As such, knife blade 174 is inhibited from being deployed when jaw members 110, 120 are disposed in the spaced-apart position.

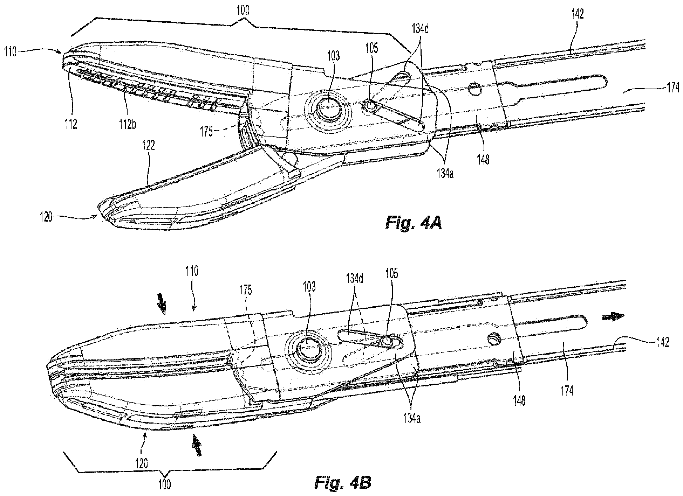

In order to move jaw members 110, 120 to the approximated position to grasp tissue therebetween, movable handle 40 is pulled proximally towards fixed handle portion 28 of housing 20 from the initial position to the compressed position (FIGS. 2A and 2B). Upon movement of movable handle 40 to the compressed position, movable handle 40 urges slider assembly 150 proximally through housing 20. Torsion spring 160, in an initial, less-tensioned state, is translated proximally together with slider assembly 150 such that upper leg 162 of torsion spring 160 pulls drive plate 142 proximally in connection with the proximal translation of slider assembly 150. At this point, slider assembly 150 and drive plate 142 move in concert with one another. As drive plate 142 is pulled proximally, cam pin 105 is pulled proximally through cam slots 134d of proximal flanges 134a of jaw members 110, 120 such that jaw members 110, 120 are pivoted from the spaced-apart position to the approximated position (FIGS. 4A and 4B) to grasp tissue therebetween.

In order to apply energy to tissue grasped between jaw members 110, 120 to treat tissue, movable handle 40 is compressed further towards fixed handle portion 28 of housing 20 to an activation position, wherein an appropriate closure force or closure force within an appropriate range, is achieved and energy activation is initiated (See FIG. 2D). As movable handle 40 is moved further proximally relative to housing 20 beyond the compressed position, an appropriate closure force or closure force within an appropriate range is imparted to tissue grasped between jaw members 110, 120 regardless of the thickness or compressibility of tissue or the position of movable handle 40. This is because, upon movement of movable handle 40 from the compressed position towards the activation position, proximal housing 152 of slider assembly 150 is translated proximally while drive plate 142 is maintained in position. Upon movement of movable handle 40 from the compressed position to the activated position, proximal housing 152 and drive plate 142 no longer move in concert with one another but are decoupled to permit relative motion therebetween.

The decoupling of proximal housing 152 of slider assembly 150 and drive plate 142 to permit relative motion therebetween is provided via torsion spring 160. More specifically, upon proximal movement of movable handle 40, a first force is imparted from movable handle 40, through proximal housing 152 of slider assembly 150, body 161 of torsion spring 160, and upper leg 162 of torsion spring 160, to drive plate 142 to urge drive plate 142 in a proximal direction, while a second, opposite force acts on drive plate 142 and, thus, upper leg 162 of torsion spring 160 in a distal direction to control the amount of compression of tissue between jaw members 110, 120. Once the second, opposite force exceeds the spring force of torsion spring 160, proximal movement of proximal housing 152 no longer results in proximal movement of drive plate 142 but, rather, results in further tensioning of torsion spring 160, wherein torsion spring 160 is wound-up, absorbing the force imparted thereto from movement of movable handle 40.

Thus, once this point has been reached, further proximal translation of proximal housing 152 of slider assembly 150 urges body 161 of torsion spring 160 proximally while upper leg 162 of torsion spring 160 remains in position as a result of the wind-up tensioning of torsion spring 160. With upper leg 162 of torsion spring 160 retained in position, drive plate 142 is likewise retained in position despite the proximal translation of movable handle 40. As such, an upper threshold of pressure applied to tissue grasped between jaw members 110, 120 is defined.

Referring to FIG. 2D, upon achieving the activation position of movable handle 40, button activation post 196 (FIG. 1) of movable handle 40 contacts depressible button 192 sufficiently so as to depress depressible button 192 into fixed handle portion 28 of housing 20 to activate switch 194. Switch 194 is disposed in electrical communication with the generator (not shown) and electrically-conductive plates 112, 122 of jaw members 110, 120 (FIG. 4A), such that activation of switch 194 initiates the supply of energy to electrically-conductive plates 112, 122 to treat, e.g., coagulate, cauterize, and/or seal, tissue grasped therebetween.

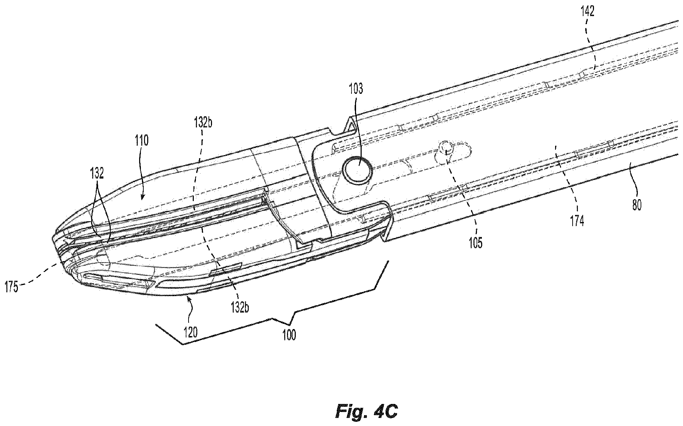

Referring to FIG. 4C, once tissue has been treated or where it is only desired to cut tissue, knife blade 174 may be advanced between jaw members 110, 120 to cut tissue grasped therebetween. In order to advance knife blade 174 from the retracted position to the extended position, trigger 72 is pulled proximally against the bias of biasing member 71 from the un-actuated position to the actuated position (FIG. 2C). As trigger 72 is pulled proximally, linkage 76 is urged to pivot counter-clockwise (compare FIG. 2C to FIG. 2D) such that upper end slot 77b of linkage 76 is moved distally. Distal movement of upper end slot 77b urges tube 78 to translate distally and, in turn, urges knife plate 172 to translate distally. This movement is permitted as proximal housing 152 is displaced relative to the movement path of lockout peg 79 with movable handle 40 in or near the compressed or actuated position.

Movement of trigger 72 from the un-actuated position to the actuated position urges knife plate 172 distally. More specifically, knife plate 172 is urged distally such that knife blade 174 is advanced distally from the retracted position to the extended position. As knife blade 174 is advanced distally, knife blade 174 extends through knife slots 112a, 112b defined within electrically-conductive plates 112, 122 to cut tissue grasped between jaw members 110, 120.

Upon release, trigger 72 and knife plate 172 are returned proximally under the bias of biasing member 71 such that knife blade 174 is returned to the retracted position. Thereafter, movable handle 40 may be released, allowing movable handle 40 to return to the initial position under the bias of lower leg 163 of torsion spring 160 abutting block 26 of housing 20, thereby returning jaw members 110, 120 to the spaced-apart position and releasing the treated and/or divided tissue.

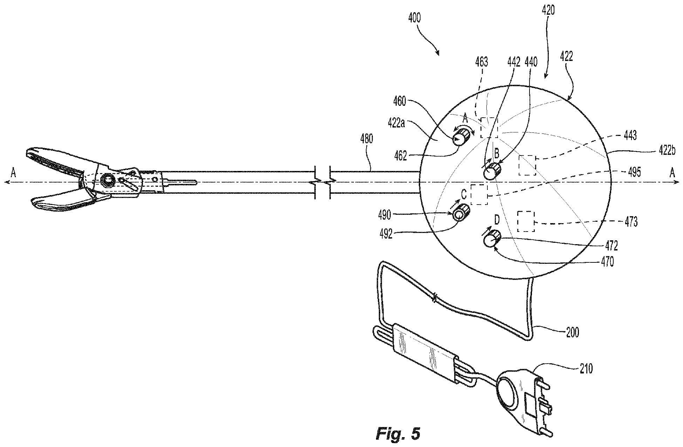

FIG. 5 shows a schematic illustration of another embodiment of a surgical instrument 400 for use with various surgical procedures. Instrument 400 includes many of the same structural and actuation elements as the aforedescribed surgical instrument 10 and only the differences will be described herein for the purposes of brevity. Moreover, the various actuators and activation elements, namely, rotation assembly 460, handle assembly 440, switch assembly 490, and trigger assembly 470 are generally shown schematically. It is contemplated that each respective assembly 460, 440, 490, and 470 may be actuated or activated using various known electrical or mechanical elements. Various mechanical and electrical actuation and activation assemblies are shown that rotate the jaw members 110, 120, actuate the jaw members 110, 120, energize the jaw members 110, 120 and cut tissue disposed between the jaw members 110, 120 and are described in commonly-owned U.S. Application Serial No. 16/244,810 filed January 10, 2019.

Instrument 400 includes a housing 420 including a rounded barrel portion 422 having a distal end portion 422a and proximal end portion 422b, the proximal end portion 422b configured to seat within a palm of a user. Similar to instrument 10, a shaft 480 extends from a distal portion of barrel portion 422 for ultimate connection to end effector assembly 100 as detailed above with respect to instrument 10. Unlike the aforedescribed instrument 10, no handle member depends from barrel portion 422, but, rather, actuation of the jaw members 110, 120 is accomplished via handle actuator assembly 440. Handle actuator assembly 440 includes an actuation element 442 disposed on an outer surface of barrel portion 422 that is configured to extend outwardly therefrom enabling manipulation by the user. In the particular embodiment shown in FIG. 5, handle actuator assembly 440 communicates with a solenoid 443 to control movement of the jaw members 110, 120 between the spaced-apart and approximated positions. See FIGS. 4A-4C. More particularly, actuation of element 442 (e.g., pressing element 442 in the direction of arrow "B" towards barrel portion 422) initiates the solenoid 443 to mechanically rotate, advance or retract a drive element (e.g., drive plate 142 of FIGS. 4A and 4B) which, in turn, pivots the jaw members 110, 120 between the spaced-apart position and the approximated position (see, e.g., FIGS. 4A and 4B) to grasp tissue therebetween.

Barrel portion 422 also supports rotation assembly 460 for manipulation by the user. Rotation assembly 460 includes rotation element 462 that is rotatable in a dial-like manner to rotate jaw members 110, 120 about longitudinal axis A-A defined through barrel portion 422 and shaft 480. More particularly, rotation of element 462 in either a clockwise or counter-clockwise direction initiates a solenoid 463 to mechanically or electro-mechanically rotate shaft 480 in the same direction. Element 462 may include a potentiometer (not shown) to regulate the speed of rotation in either direction.

Barrel portion 422 also supports knife actuation assembly 470 for manipulation by the user. Knife actuation assembly 470 includes an actuation element 472 disposed on an outer surface of barrel portion 422 that is configured to extend outwardly therefrom enabling manipulation by the user. More particularly, actuation of element 472 (e.g., pressing element 472 in the direction of arrow "D" towards barrel portion 422) initiates a solenoid 473 to mechanically rotate, advance or retract a knife drive element (e.g., knife drive plate 172 of FIGS. 4A and 4B)) which, in turn, advances and retracts the knife blade 175 between the jaw members 110, 120 (see, e.g., FIGS. 4B and 4C) to cut tissue therebetween. It is contemplated that element 472 may alternatively be an electrical activation switch and, when actuated, configured to energize a cutting element disposed between jaw members 110, 120.

Barrel portion 422 also supports energy activation assembly 490 for manipulation by the user. Energy activation assembly 490 includes an actuation element 492 disposed on an outer surface of barrel portion 422 that is configured to extend outwardly therefrom enabling manipulation by the user. More particularly, actuation of element 492 (e.g., pressing element 492 in the direction of arrow "C" towards barrel portion 422) initiates a switch 493 that energizes jaw members 110, 120 to treat, e.g., seal, tissue disposed between jaw members 110, 120.

As mentioned above, instrument 400 also includes electrosurgical cable 200 including a proximal plug 210 that connects instrument 400 to a generator (not shown) or other suitable power source. Electrosurgical cable 200 includes lead wires extending therethrough that have sufficient length to extend through housing 420 and shaft 480 in order to operably couple the generator, energy activation assembly 490, and end effector assembly 100 with one another to enable the selective supply of energy to jaw members 110, 120 of end effector assembly 100, e.g., upon activation of activation element 492 of energy activation assembly 490.

Activation element 492 of energy activation assembly 490 mechanically couples to a switch 495 mounted within barrel portion 422 that is configured to electrically communicate with end effector assembly 100 and the generator (not shown) via suitable electrical wiring (not shown) to enable energy to be supplied from the generator (not shown) to end effector assembly 100 upon activation of switch 495. Switch 495 may include tactile or audible elements to provide feedback to the user prior to, during, or after activation and treatment. One or more safety features 493 may be mechanically, electromechanically or electrically utilized to limit deployment of the knife blade 175 to cut tissue. For example, element 472 may be electrically or mechanically (or a combination thereof) impeded from activation prior to completion of tissue treatment, e.g., successful completion of a tissue seal.

All of the various assemblies 440, 460, 470, and 490 and respective activation elements 442, 462, 472, and 492 and solenoids 443, 463, 473 or switches 495 may be utilized in various combinations and at various locations about the outer surface of barrel portion 422. Moreover, any of the assemblies 440, 460, 470, and 490 may be configured to be actuated/activated via one or a combination of mechanical or electrical movements, e.g., depressible/extendible, rotatable, toggle or slidable, or variations thereof.

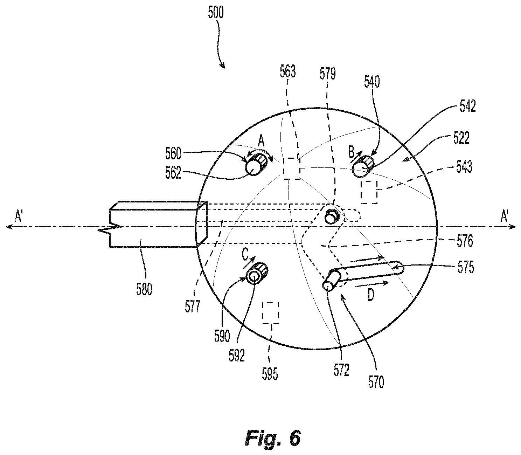

FIG. 6 shows a schematic illustration of another embodiment of a surgical instrument 500 for use with various surgical procedures. Instrument 500 includes many of the same structural and actuation elements as the aforedescribed surgical instruments 10 and 400 and only the differences will be described herein for the purposes of brevity. Moreover, the various actuators and activation elements, namely, rotation assembly 560, handle assembly 540, switch assembly 590, and trigger assembly 570 are generally shown schematically. It is contemplated that each respective assembly 560, 540, 590, and 570 may be actuated or activated using various known electrical or mechanical elements. Various mechanical and electrical actuation and activation assemblies are shown and are described in commonly-owned U.S. Application Serial No. 16/244,810 filed January 10, 2019.

Moreover, the location of the various assemblies 560, 540, 590, and 570 on barrel portion 522 may be interchanged or relocated depending upon a particular purpose. As such, the locations of these assemblies 560, 540, 590, and 570 is generalized.

Instrument 500 includes a housing 520 having rounded barrel portion 522. Similar to instruments 10 and 400, a shaft 580 extends from a distal portion of barrel portion 522 for ultimate connection to end effector assembly 100 as detailed above with respect to instrument 10. Handle actuator assembly 540 includes an actuation element 542 disposed on an outer surface of barrel portion 522 and is configured to extend outwardly therefrom enabling manipulation by the user. Handle actuator assembly 540 communicates with a solenoid 543 to control movement of the jaw members 110, 120 between the spaced-apart and approximated positions. See FIGS. 4A-4C. More particularly, actuation of element 542 (e.g., pressing element 542 in the direction of arrow "B" towards barrel portion 522) initiates the solenoid 543 to mechanically rotate, advance or retract a drive element (e.g., drive plate 142 of FIGS. 4A and 4B) which, in turn, pivots the jaw members 110, 120 between the spaced-apart position and the approximated position (see, e.g., FIGS. 4A and 4B) to grasp tissue therebetween.

Barrel portion 522 also supports rotation assembly 560 for manipulation by the user. Rotation assembly 560 includes rotation element 562 that is rotatable in a dial-like manner to rotate jaw members 110, 120 about longitudinal axis A'-A' defined through barrel portion 522 and shaft 580. More particularly, rotation of element 562 in either a clockwise or counter-clockwise direction initiates a solenoid 563 to mechanically or electro-mechanically rotate shaft 580 in the same direction. Element 562 may include a potentiometer (not shown) to regulate the speed of rotation in either direction.

Barrel portion 522 also supports knife actuation assembly 570 for manipulation by the user. Knife actuation assembly 570 includes an actuation element 572 disposed on an outer surface of barrel portion 522 and is configured to slide along a channel 575 defined in barrel portion 522. More particularly, actuation of element 572 (e.g., sliding proximally in the direction of arrow "D") pivots a link 576 about a pivot 579 disposed within barrel 522 which, in turn, urges a knife drive element 577 distally to advance the knife 175 through tissue disposed between jaw members 110, 120 (see FIGS. 2A-4B). Alternatively, a solenoid (not shown) may be configured to mechanically rotate, advance or retract a knife drive element 577 (similar to knife drive plate 172 of FIGS. 4A and 4B) which, in turn, advances and retracts the knife blade 175 between the jaw members 110, 120 (see, e.g., FIGS. 4B and 4C) to cut tissue therebetween. Other known mechanical and electrical (or combinations thereof) are also contemplated for advancing the knife 175. A spring (not shown) may be configured to bias the knife drive element 577 in a proximal-most position.

Barrel portion 522 also supports energy activation assembly 590 for manipulation by the user. Energy activation assembly 590 includes an actuation element 592 disposed on an outer surface of barrel portion 522 and is configured to extend outwardly therefrom enabling manipulation by the user. More particularly, actuation of element 592 (e.g., pressing element 592 in the direction of arrow "C" towards barrel portion 522) initiates a switch 595 that energizes jaw members 110, 120 to treat, e.g., seal, tissue disposed between jaw members 110, 120.

Similar to instruments 10 and 400, instrument 500 also includes electrosurgical cable 200 including a proximal plug 210 that connects instrument 500 to a generator (not shown) or other suitable power source.

Activation element 592 of energy activation assembly 590 mechanically couples to switch 595 mounted within barrel portion 522 that is configured to electrically communicate with end effector assembly 100 and the generator (not shown) via suitable electrical wiring (not shown) to enable energy to be supplied from the generator (not shown) to end effector assembly 100 upon activation of switch 595. Switch 595 may include tactile or audible elements to provide feedback to the user prior to, during, or after activation and treatment. Similar to switch assembly 490, one or more safety features may be mechanically, electromechanically or electrically utilized with switch assembly 590 to limit deployment of the knife blade 175 to cut tissue.

All of the various assemblies 540, 560, 570, and 590 and respective activation elements 542, 562, 572, and 592 and solenoids 543, 563 or switches 595 may be utilized in various combinations and at various locations about the outer surface of barrel portion 522. Moreover, any of the assemblies 540, 560, 570, and 590 may be configured to be actuated and activated via one or a combination of mechanical or electrical movements, e.g., depressible/extendible, rotatable, toggle or slidable or variations thereof.

The various embodiments disclosed herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

From the foregoing and with reference to the various figure drawings, those skilled in the art will appreciate that certain modifications can also be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A surgical instrument, comprising:
   a housing having a rounded barrel portion with a distal end portion and proximal end portion configured to seat within a palm of a user;
   a shaft extending from the distal end portion of the barrel portion, the shaft supporting an end effector assembly at a distal end thereof configured to treat tissue, the end effector assembly including first and second jaw members;
   a rotating assembly disposed on an outer surface of the barrel portion and actuatable to rotate the jaw members about a longitudinal axis defined through the shaft;
   a jaw actuation assembly disposed on the outer surface of the barrel portion and actuatable to move the jaw members between an open position wherein the jaw members are spaced relative to one another for manipulating tissue and a closed position for approximating tissue therebetween;
   an energy activation assembly disposed on the outer surface of the barrel portion and actuatable to supply electrosurgical energy from an electrosurgical energy source to the jaw members upon activation thereof; and
   a knife actuation assembly disposed on the outer surface of the barrel portion and actuatable to cut tissue disposed between the jaw members upon actuation thereof.
2. The surgical instrument according to paragraph 1, wherein at least one of the rotating assembly, jaw actuation assembly, or knife actuation assembly includes a solenoid to facilitate actuation.
3. The surgical instrument according to paragraph 1, wherein at least one of the rotating assembly, jaw actuation assembly, or knife actuation assembly is a dial.
4. The surgical instrument according to paragraph 1, wherein at least one of the rotating assembly, jaw actuation assembly, energy activation assembly or knife actuation assembly is a depressible button.
5. The surgical instrument according to paragraph 1, wherein at least one of the rotating assembly, jaw actuation assembly, or knife actuation assembly is a slide actuator.
6. The surgical instrument according to paragraph 1, wherein the energy activation assembly electrically communicates with a switch to energize the jaw members.
7. The surgical instrument according to paragraph 1, wherein the energy activation assembly includes a safety that impedes actuation of the knife actuation assembly until after successful completion of a tissue seal.
8. The surgical instrument according to paragraph 1, wherein the rotating assembly includes a potentiometer for regulating the speed of rotation of the jaw members about the longitudinal axis.
9. A surgical instrument, comprising:
   a housing having a rounded barrel portion with a distal end portion and proximal end portion configured to seat within a palm of a user;
   a shaft extending from the distal end portion of the barrel portion, the shaft supporting an end effector assembly at a distal end thereof configured to treat tissue, the end effector assembly including first and second jaw members;
   a rotating assembly disposed on an outer surface of the barrel portion and actuatable to rotate the jaw members about a longitudinal axis defined through the shaft;
   a jaw actuation assembly disposed on the outer surface of the barrel portion and actuatable to move the jaw members between an open position wherein the jaw members are spaced relative to one another for manipulating tissue and a closed position for approximating tissue therebetween;
   an energy activation assembly disposed on the outer surface of the barrel portion and actuatable to supply electrosurgical energy from an electrosurgical energy source to the jaw members upon activation thereof; and
   a knife actuation assembly disposed on the outer surface of the barrel portion and actuatable to cut tissue disposed between the jaw members upon actuation thereof, the knife actuation assembly including a knife blade and an actuation element, the actuation element coupled to a first end of a linkage and a knife drive element couple to a second end of the linkage, wherein actuation of the activation element pivots the linkage to force the knife drive element distally to translate the knife blade through tissue disposed between the jaw members.
10. The surgical instrument according to paragraph 9, wherein at least one of the rotating assembly or jaw actuation assembly includes a solenoid to facilitate actuation.
11. The surgical instrument according to paragraph 9, wherein at least one of the rotating assembly or jaw actuation assembly is a dial.
12. The surgical instrument according to paragraph 9, wherein at least one of the rotating assembly, jaw actuation assembly, or energy activation assembly is a depressible button.
13. The surgical instrument according to paragraph 9, wherein the energy activation assembly electrically communicates with a switch to energize the jaw members.
14. The surgical instrument according to paragraph 9, wherein the energy activation assembly includes a safety that impedes actuation of the knife actuation assembly until after successful completion of a tissue seal.
15. The surgical instrument according to paragraph 9, wherein the rotating assembly includes a potentiometer for regulating the speed of rotation of the jaw members about the longitudinal axis.

## Claims

1. A surgical instrument, comprising:
a housing having a rounded barrel portion with a distal end portion and proximal end portion configured to seat within a palm of a user;
a shaft extending from the distal end portion of the barrel portion, the shaft supporting an end effector assembly at a distal end thereof configured to treat tissue, the end effector assembly including first and second jaw members;
a rotating assembly disposed on an outer surface of the barrel portion and actuatable to rotate the jaw members about a longitudinal axis defined through the shaft;
a jaw actuation assembly disposed on the outer surface of the barrel portion and actuatable to move the jaw members between an open position wherein the jaw members are spaced relative to one another for manipulating tissue and a closed position for approximating tissue therebetween;
an energy activation assembly disposed on the outer surface of the barrel portion and actuatable to supply electrosurgical energy from an electrosurgical energy source to the jaw members upon activation thereof; and
a knife actuation assembly disposed on the outer surface of the barrel portion and actuatable to cut tissue disposed between the jaw members upon actuation thereof.

2. The surgical instrument according to claim 1, wherein at least one of the rotating assembly, jaw actuation assembly, or knife actuation assembly includes a solenoid to facilitate actuation.

3. The surgical instrument according to claim 1 or 2, wherein at least one of the rotating assembly, jaw actuation assembly, or knife actuation assembly is a dial.

4. The surgical instrument according to claim 1,2 or 3 wherein at least one of the rotating assembly, jaw actuation assembly, energy activation assembly or knife actuation assembly is a depressible button.

5. The surgical instrument according to any preceding claim, wherein at least one of the rotating assembly, jaw actuation assembly, or knife actuation assembly is a slide actuator.

6. The surgical instrument according to any preceding claim, wherein the energy activation assembly electrically communicates with a switch to energize the jaw members.

7. The surgical instrument according to any preceding claim, wherein the energy activation assembly includes a safety that impedes actuation of the knife actuation assembly until after successful completion of a tissue seal.

8. The surgical instrument according to any preceding claim, wherein the rotating assembly includes a potentiometer for regulating the speed of rotation of the jaw members about the longitudinal axis.

9. A surgical instrument, comprising:
a housing having a rounded barrel portion with a distal end portion and proximal end portion configured to seat within a palm of a user;
a shaft extending from the distal end portion of the barrel portion, the shaft supporting an end effector assembly at a distal end thereof configured to treat tissue, the end effector assembly including first and second jaw members;
a rotating assembly disposed on an outer surface of the barrel portion and actuatable to rotate the jaw members about a longitudinal axis defined through the shaft;
a jaw actuation assembly disposed on the outer surface of the barrel portion and actuatable to move the jaw members between an open position wherein the jaw members are spaced relative to one another for manipulating tissue and a closed position for approximating tissue therebetween;
an energy activation assembly disposed on the outer surface of the barrel portion and actuatable to supply electrosurgical energy from an electrosurgical energy source to the jaw members upon activation thereof; and
a knife actuation assembly disposed on the outer surface of the barrel portion and actuatable to cut tissue disposed between the jaw members upon actuation thereof, the knife actuation assembly including a knife blade and an actuation element, the actuation element coupled to a first end of a linkage and a knife drive element couple to a second end of the linkage, wherein actuation of the activation element pivots the linkage to force the knife drive element distally to translate the knife blade through tissue disposed between the jaw members.

10. The surgical instrument according to claim 9, wherein at least one of the rotating assembly or jaw actuation assembly includes a solenoid to facilitate actuation.

11. The surgical instrument according to claim 9 or 10, wherein at least one of the rotating assembly or jaw actuation assembly is a dial.

12. The surgical instrument according to claim 9, 10 or 11 wherein at least one of the rotating assembly, jaw actuation assembly, or energy activation assembly is a depressible button.

13. The surgical instrument according to any one of claims 9 to 12 wherein the energy activation assembly electrically communicates with a switch to energize the jaw members.

14. The surgical instrument according to any one of claims 9 to 13, wherein the energy activation assembly includes a safety that impedes actuation of the knife actuation assembly until after successful completion of a tissue seal.

15. The surgical instrument according to any one of claims 9 to 14 , wherein the rotating assembly includes a potentiometer for regulating the speed of rotation of the jaw members about the longitudinal axis.
